# EUROPEAN PATENT APPLICATION

(11) **EP 0 953 569 A1**
(43) Date of publication of application: **03.11.1999**
(21) Application number: 98201399.7
(22) Date of filing: 29.04.1998
(51) Int. Cl.: C07D 499/12, C07D 501/06

(54) **Preparation of beta-lactam antibiotics in the presence of urea or amide**

(71) Applicant: GIST-BROCADES B.V., 2600 MA Delft (NL)
(72) Inventor: Leenderts, Everardus Johannes Anthonius Maria, 3161 LL Rhoon (NL); El-Sayad, Hassan Aly, 8th District Nasr City, Cairo (EG); Walraven, Hubertus Gerardus Maria, 2284 TM Rijswijk (NL); Wesseling, Jolanda, 3039 RJ Rotterdam (NL)
(74) Representative: Visser-Luirink, Gesina, Dr.

(57) **Abstract**

A process for the preparation of β-lactam compounds has been described wherein before the addition of a base, urea or derivatives thereof and/or amide or derivatives has been added to (a) before an acid hydrolysis of the Dane salt coupling reaction mixture resulting from acylation of 6-amino-penicillanic acid, 7-amino-cephalosporanic acid or 7-amino-3'-desacetoxycephalosporanic acid and derivatives thereof at low temperature or to (b) a solution obtained by the addition of an acid to the crude β-lactam antibiotic compound in water and/or one or more organic solvents.

## Description

The present invention relates to an improved process for the preparation of β-lactam antibiotic compounds in the presence of urea or derivatives thereof and/or amide or derivatives thereof.

It is commonly accepted that the most economical approach for the isolation of compounds such as β-lactam antibiotics or their intermediates from their solutions comprising the same is crystallization.

Various processes have been proposed for the preparation of the semi-synthetic β-lactam antibiotic compounds such as penicillins and cephalosporins. These proposals generally involve the acylation of 6-aminopenicillanic acid (6-APA) or 7-aminocephalosporanic acid (7-ACA) or a protective derivative thereof using, for example, an acyl halide serving to introduce the desired acyl group. For example in European patent application EP 0011513, a process has been described for the preparation of zwitterionic antibiotics, for instance, ampicillin, amoxicillin or cephalexin through acylation of silylated 6-aminopenicillanic acid or 7-aminocephalosporanic acid with a phenylglycyl halide or p-hydroxyphenylglycyl halide in the presence of N-alkylpyrrolidone as an acid acceptor. Similarly United States patent US 4,248,780 for example, describes the preparation of zwitterionic antibiotic ampicillin through acylation of silylated 6-aminopenicillanic acid with a phenylglycylchloride hydrochloride by using urea as a base.

Furthermore, a number of patent applications and patents disclose preparation method of α-aminoacylpenicillanic acid derivatives by acylating 6-APA with mixed anhydrides derived from modified Dane salts of D-2-amino-(p-hydroxyphenyl)-acetic acid, such as described in German patent Applications Ser. No. 1,302,847, No. 2,020,133 and No. 2,065,879 and British Patents No. 1,327,270 and No. 1,347,979.

United States patent US 4,358,588 describes an improved process for the preparation of D-α-amino-p-hydroxyphenyl-acetamide-penicillanic acid and cephalosporanic acid derivatives by reacting an appropriate mixed anhydride of a Dane salt with a protected derivative, for example 6-APA or 7-ACA in the presence of a tertiary amine. This type of acylation is referred to as Dane salt coupling reaction.

Thereafter, the β-lactam antibiotic compounds are isolated from an acidic aqueous solution, obtained by acidic hydrolysis of the reaction mixture after the Dane salt coupling reaction, by adding a base and followed by filtration.

A major drawback of the processes for the preparation of β-lactam antibiotic compounds is the crystallization of unwanted by-products, among which the hydrochloric acid salts of the β-lactam antibiotic compounds are the most notorious one. These unwanted crystallization of by-products can occur at any stage of the hydrolysis or crystallization procedure.

The formation of acid salts of the β-lactam antibiotic compounds is strongly dependent upon temperature, pH and time during the acidic hydrolysis of the Dane salt coupling reaction. Lower temperatures, a lower pH and prolonged time during the hydrolysis of the Dane salt coupling reaction result in the production of an increased amount of acid salt of the corresponding β-lactam antibiotic compound. At higher temperatures, however, the formation of the corresponding acid salt of the β-lactam during hydrolysis of the Dane salt coupling reaction is reduced but on the other hand the cleavage of the β-lactam is enhanced. Also, the presence of seeding crystals corresponding to acid salt of the β-lactam compound, produced during the hydrolysis of the Dane salt coupling reaction, facilitate the formation of the acid salts of the β-lactam antibiotic compounds. Especially the formation of the seeding material during the hydrolysis of Dane coupling reaction is enhanced on an industrial scale process compared to a laboratory scale process. Thus, the crystalline products resulting from the Dane salt coupling reaction often contain unacceptable levels of impurities.

It was found surprisingly that β-lactam antibiotics free of acid salts of the corresponding β-lactams can be prepared under addition of urea or derivatives thereof and/or amide or derivatives thereof after the Dane salt coupling reaction at low temperature. Urea or derivatives thereof and/or amide or derivatives thereof can also be added during the purification of crude β-lactam antibiotic compounds during the crystallization phase. Besides the lower content of the hydrochloric acid salt of the β-lactam produced, these processes also result in a higher yield of the end product. This method has nowhere been described or suggested for β-lactam compounds until now.

### Summary of the invention

The present invention provides an improved process for the preparation of β-lactam antibiotic compounds in the presence of urea or derivatives thereof and/or amide or derivatives thereof.

The β-lactam compounds are crystallized by the addition of a base to a solution of β-lactam compound obtained by
(a) an acylation reaction of 6-amino-penicillanic acid, 7-amino-cephalosporanic acid, 7-amino-3'-desacetoxycephalosporanic acid or 3-chloro-7-aminodesacetoxydesmethylcephalosporanic acid and derivatives thereof with a mixed-anhydride of the Dane salt of D-phenyl-glycine, D-p-hydroxyphenyl-glycine or D-2(1,4-cyclohexadien-1-yl)glycine, followed by acid hydrolysis of the resulting reaction mixture at low temperature or
(b) an acidic solution of a crude β-lactam compound in water and/or one or more organic solvents,
   characterised by addition of urea or derivatives thereof and/or amide or derivatives thereof with the formula (A) and (B) respectively, 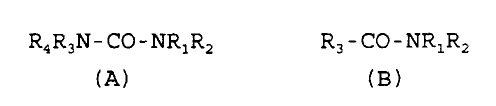 wherein R₁, R₂, R₃ and R₄ are each independently chosen from the group consisting of hydrogen, lower alkyl and allyl, or R₂ and R₃ form -CH₂-CH₂-, -CH₂-CH₂-CH₂-, -CH₂-CO- and -CH=CH-CO-resulting together with -N-CO-N- in a five or six membered cyclic ring,
   and preferably R₁, R₂, R₃ and R₄R are each chosen from hydrogen or methyl, and more preferably R₁, R₂, R₃ and R₄R are each hydrogen,
   wherein in case of process (a) urea or derivatives thereof and/or amide or derivatives thereof are added after the Dane salt coupling reaction step.

The amount of urea or derivatives thereof and/or amide or derivatives thereof present during the crystallization process is between 0.001 mol/mol β-lactam and 7.5 mol/mol β-lactam, preferably between 0.1 mol/mol β-lactam and 5.25 mol/mol β-lactam.

Before starting the crystallization process through the addition of a base, the pH of the hydrolysis mixture is maintained normally between 0.1 and 3.5 and preferably between 0.3 and 2.5 depending on the type of β-lactam compound to be produced by the addition of an acid. The temperature of the hydrolysis is between -50°C and 10°C, preferably between -5°C and 5°C and most preferably between -5°C and 0°C. The acid is preferably an inorganic acid such as hydrochloric acid or nitric acid or sulphuric acid.

Suitably the crystallization temperature is between -10°C and 50°C, preferably between -5°C and 35°C. It will be clear for the skilled in the art that the preferred conditions will depend on the β-lactam compound to be prepared.

The β-lactam antibiotic compounds can be isolated in a conventional manner, by adjusting the pH to the isoelectric point of the corresponding β-lactam compounds.

The β-lactam compounds are for instance penicillins and cephalosporins. Examples of penicillins are amoxicillin, ampicillin and epicillin. Examples of cephalosporins are cefaclor, cefadroxil, cefetamet, cefotaxim, cephalexin, cephaloglycin, cephradine and cefroxadine.

### Detailed description of the invention

The process for the preparation of crystalline β-lactam antibiotic compounds according to the present invention comprises
a) a reaction mixture in which urea or derivatives thereof and/or amide or derivatives thereof is added after the Dane salt coupling reaction of 6-amino-penicillanic acid (6-APA), 7-amino-cephalosporanic acid (7 -ACA), 7-amino-3'-desacetoxycephalosporanic acid (7-ADCA) or 3-chloro-7-aminodesacetoxydesmethylcephalosporanic acid (7-ACCA) and derivatives thereof with a reactive Dane mixed-anhydride at low temperature and
b) a solution obtained by the addition of an acid to the crude β-lactam antibiotic compound in water and/or one or more organic solvents comprising urea or derivatives thereof and/or amide or derivatives thereof,
   followed by the addition of a base.

By derivatives of 6-APA, 7-ACA, 7-ADCA and 7-ACCA have been meant salts and esters of the corresponding β-lactams.

The Dane salt coupling reaction is carried out in a dry water-insoluble organic solvent, for instance dichloromethane. Preferably, a small amount of a (C₁ - C₆)alkanol, specifically methanol, ethanol, 1-propanol, 2-propanol, 1-butanol, 2-butanol, 2-methyl-1-propanol, or a ketone, specifically acetone, methylethylketone, methylisobutylketone or an ester, specifically methyl acetate, ethyl acetate, isopropyl acetate, butyl acetate or a mixture thereof as co-solvent is added after the Dane salt coupling reaction. The concentration of the co-solvent is between 0.1% and 60%, preferably between 25% and 60% depending on the type of the β-lactam to be prepared. In case of process (a) the co-solvents are added after the Dane salt coupling reaction.

The pH of the hydrolysis solution is maintained between 0.1 and 3.5, preferably between 0.3 and 2.5 by using an acid at a low temperature between -50°C and 10°C, preferably between -5°C and 5°C and more preferably between -5°C and 0°C. At higher temperatures, the cleavage of the sensitive β-lactam ring of the antibiotic compound is enhanced.

The acid is an inorganic acid, preferably hydrochloric acid, sulphuric acid, nitric acid, phosphoric acid or an organic acid, preferably formic acid and suitable acetic acid or derivatives thereof. More preferably, hydrochloric acid or nitric acid or sulphuric acid is used.

The β-lactam antibiotic compound is isolated in conventional manner, by adjusting pH between 2.0 and 7.0, preferably to the isoelectric point of the said β-lactam antibiotic compound by addition of a base.

Furthermore, seeding material is optionally added to the crystallization solution before the addition of the base.

The base is an inorganic base, preferably sodium hydroxide, potassium hydroxide, ammonium hydroxide or an organic base, preferably an amine.

Accordingly, after filtration and drying crystalline products substantially free of impurities such as the acid salt of the same, β-lactam compounds are obtained whereas known procedures without application of urea or derivatives thereof and/or amide or derivatives thereof give higher levels of impurity, especially when the crystallization is performed at a lower temperature.

The process of the present invention is particularly suitable for the preparation of β-lactam antibiotics in the form of a zwitterionic amino acid. Examples of said antibiotics are amoxicillin, ampicillin, epicillin, cefaclor, cefadroxil, cefetamet, cefotaxim, cephalexin, cephaloglycin, cephradine and cefroxadine.

According to a further aspect of the present invention the β-lactam compound is advantageously crystallized by simultaneous addition of the β-lactam solution and a base to a crystallization vessel as described in non-published European patent application No. 97203484.7. This crystallization process comprises simultaneous addition of a β-lactam compound solution containing urea or derivatives thereof and/or amide or derivatives thereof and a titrant, namely the base, to a crystallization vessel.

According to another procedure the β-lactam compound solution and the titrant (base) is added simultaneous to the crystallization vessel containing urea or derivatives thereof and/or amide or derivatives thereof.

The process of crystallization of antibiotic compounds, according to the present invention, has a major advantage over the existing methods because better concordance results are possible. The concordance value related to the difference between titration with a strong acid and titration with a strong base has been defined according to the United States Pharmacopoeia, twenty-first revision, dated January 1, 1985. A small concordance value stands for a constant quality product. Ampicillin trihydrate, for instance isolated from crystallization of a solution containing ampicillin hydrochloride has a concordance value of less than 1 mol %, while crystallization through a prior art procedure of a number of experiments has given a concordance value between 4 and 8 mol %. The β-lactam antibiotic compounds with a small concordance value, i.e. comprising lower amounts of acid salt of the corresponding β-lactam antibiotics, are suitable for pharmaceutical formulation purposes.

Furthermore, the bad effect of storage and temperature (higher than room temperature) on the stability of the β-lactam antibiotic compound is enhanced due to the presence of very small or negligible amounts of the corresponding acid salt of β-lactam antibiotic compound. These acid salts of the β-lactam antibiotic are usually hygroscopic in nature.

The invention will now be described with reference to the following Example, which are not to be constructed as being limiting on the invention, and are provided purely for illustrative purposes.

### Example

STEP A: Preparation of methoxycarbonyl D-α-(1-carbomethoxypropen-2-yl) amino-p-hydroxyphenyl-acetate. 85 ml of N,N-dimethylacetamide, 116.4 g of potassium D-α-[1-(carbomethoxypropen-2-yl)-2-amino]-phenylacetate and 0.3 ml pyridine were added to 267 ml of dichloromethane and the resulting suspension was cooled to -40°C. Subsequently 46.3 g of pivaloylchloride were added to this solution. While maintaining a temperature of -30°C the mixture was stirred for 90 minutes and then cooled to -50°C.
STEP B: 63.8 g of triethylamine were added to a suspension of 80.0 g of 6-aminopenicillanic acid in 533 ml of dichloromethane at 20°C and the mixture was stirred for 90 minutes. The mixture should contain enough water for obtaining a clear solution. At 5°C, 78 g of 2-ethylhexanoic acid were added and the solution was further cooled to -50°C.
STEP C: Preparation of 6-(D-α-amino-p-hydroxy-phenylacetamido)-penicillanic acid.
The reaction mixture obtained in STEP A was added in about 3 minutes into the solution obtained in STEP B with vigorous stirring. In 3 hours the temperature was gradually increased from -40°C / -35°C to -10°C / -5°C. Subsequently, a solution of urea (100 g in 800 ml of demineralised water) was added. While maintaining the mixture at between -5°C and 0°C concentrated hydrochloric acid was added and hydrolysis was continued for 30 minutes at a pH between 1.4 and 1.5. The organic phase was separated and the product was crystallised from the aqueous layer by addition of approximately 45 ml of 8 N sodium hydroxide solution until pH between 4.8 and 5.0 at a temperature of between 2°C and 5°C. The suspension was kept over night under these conditions, filtered, washed with 100 ml of chilled, demineralised water and 300 ml of chilled acetone. The cake was dried at 35°C till a constant weight was reached to obtaine about 143 g of amoxicillin trihydrate with a purity of 86.1% as amoxicillin anhydrous (HPLC).

## Claims

1. A process to prepare a crystalline β-lactam compound by the addition of a base to a solution of a β-lactam compound obtained by
(a) an acylation reaction of 6-amino-penicillanic acid, 7-amino-cephalosporanicacid, 7-amino-3'-desacetoxycephalosporanicacid or 3-chloro-7-aminodesacetoxydesmethylcephalosporanic acid or derivatives thereof with a mixed-anhydride of the Dane salt of any one of the compound comprising D-phenyl-glycine, D-p-hydroxyphenyl-glycine or D-2(1,4-cyclohexadien-1-yl)glycine in one or more organic solvents, followed by acid hydrolysis of the resulting reaction mixture or
(b) an acidic solution of a crude β-lactam compound in water and/or one or more organic solvents,
characterised by the addition of urea or derivatives thereof and/or amide or derivatives thereof with the formula (A) and (B) respectively, 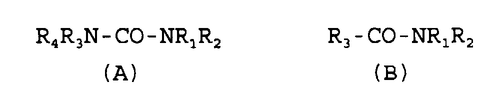 wherein R₁, R₂, R₃ and R₄ are each independently chosen from the group consisting of hydrogen, lower alkyl and allyl, or R₁, and R₃ form -CH₂-CH₂-, -CH₂-CH₂-CH₂-, -CH₂-CO- and -CH=CH-CO-resulting together with -N-CO-N- in a five or six membered cyclic ring, (C₁ - C₆)alkanol esters of (C₁ - C₆)carboxylic acid.

2. A process according to claim 1 wherein in the formula (A), R¹, R², R³ and R⁴ are each chosen from hydrogen or methyl.

3. A process according to claims 1 or 2, wherein when applying process (a) urea or derivatives thereof and/or amide or derivatives thereof are added after the coupling reaction.

4. A process according to any one of the claims 1-3, wherein when applying process (a), the hydrolysis temperature is maintained between -50°C and 10°C.

5. A process according to claims 1-4, wherein the amount of urea or derivatives thereof and/or amide or derivatives thereof is between 0.001 mol/mol β-lactam and 7.5 mol/mol β-lactam, preferably between 0.1 mol/mol β-lactam and 5.25 mol/mol β-lactam.

6. A process according to claims 1-5, wherein the solution of the β-lactam antibiotic compound comprises one or more of the co-solvents (C₁ - C₆)alkanol or ketone or (C₁ - C₆)carboxylates with a (C₁ - C₆)alkanol.

7. A process according to claim 6, wherein when applying process (a) the co-solvents are added after the coupling reaction.

8. A process according to claim 6 or 7, wherein the concentration of (C₁ - C₆)alkanol or ketone or ester is between 0.1% and 60%.

9. A process according to claims 1-8, wherein the pH of the acidic solution has been maintained between 0.1 and 3.5, preferably between 0.3 and 2.5 by using an acid.

10. A process according to claim 9, wherein the acid is an inorganic acid, preferably hydrochloric acid or nitric acid or sulphuric acid.

11. A process according to claims 1-10 wherein, during crystallization, the pH is maintained between 2.0 and 7.0, preferably close to the isoelectric point of said β-lactam compound by addition of a base.

12. A process according to claims 1-11 wherein the base is an inorganic base, preferably sodium hydroxide, potassium hydroxide or ammonium hydroxide.

13. A process according to any one of the claim 12 wherein seeding crystals are added before the addition of the base.

14. A process according to any one of the claims 1-13 wherein the temperature during the crystallization is maintained between -10°C and 50°C, preferably between -5°C and 35°C.

15. A process according to any one of the claims 1-14 wherein the β-lactam compounds are penicillins and cephalosporins, preferably amoxicillin, ampicillin, epicillin, cefaclor, cefadroxil, cefetamet, cefotaxim, cephalexine, cephaloglycin, cephradine and cefroxadine.

16. A process to prepare a crystalline β-lactam compound by simultaneous addition of the β-lactam compound solution or derivatives thereof and a base to a crystallization vessel, characterized by the presence of urea or derivatives thereof and/or amide or derivatives thereof in the solution of the β-lactam compound.

17. A β-lactam compound obtainable by a method according to any of the preceding claims.
